# EUROPEAN PATENT SPECIFICATION

(11) **EP 4 153 562 B1**
(45) Date of publication and mention of the grant of the patent: **09.07.2025**
(21) Application number: 21724717.0
(22) Date of filing: 17.05.2021
(51) Int. Cl.: C07C 221/00, C07C 225/22

(54) **PROCESS FOR PURIFYING 2-(4'-DIETHYLAMINO-2'-HYDROXYBENZOYL)BENZOIC ACID HEXYL ESTER**
VERFAHREN ZUR REINIGUNG VON 2-(4'-DIETHYLAMINO-2'-HYDROXYBENZOYL)BENZOESÄUREHEXYLESTER
PROCÉDÉ DE PURIFICATION D'ESTER D'HEXYLE D'ACIDE 2-(4'-DIÉTHYLAMINO-2'-HYDROXYBENZOYL)BENZOÏQUE

(30) Priority: 19.05.2020 EP 20175409
(43) Date of publication of application: 29.03.2023
(73) Proprietor: BASF SE, 67056 Ludwigshafen am Rhein (DE)
(72) Inventor: BINDER, Axel, 67056 Ludwigshafen (DE); KRONEMAYER, Helmut, 67056 Ludwigshafen (DE); SCHEIN-ALBRECHT, Karin, 67056 Ludwigshafen (DE); BLANCHOT, Mathieu, 67056 Ludwigshafen (DE); LANG, Rieke, 67056 Ludwigshafen (DE); EHLIS, Thomas, 4133 Schweizerhalle (Muttenz) (CH)
(74) Representative: BASF IP Association
(86) International application number: PCT/EP2021/062932
(87) International publication number: WO 2021/233805

(56) References cited:
- WO-A1-03/097578

## Description

The present invention relates to a process for purifying 2-(4'-diethylamino-2'-hydroxybenzoyl)benzoic acid hexyl ester providing a high purity and low phthalic acid dialkyl ester content.

UV radiation causes harmful effects on the human skin. Beside the acute effect of sunburn of the skin, UV radiation is also known to increase the risk of skin cancer. Furthermore, long time exposure to UV-A and UV-B light can cause phototoxic and photo allergenic reactions on the skin and can accelerate skin aging.

To protect the human skin from UV radiation, various sun protecting UV filters (also referred to as UV absorbers) exist including UV-A filter, UV-B filter, and broadband filters. These filters are added to sunscreen or cosmetic compositions. The UV filters are either organic or inorganic, particulate or non-particulate compounds, of which all have a high absorption efficacy in the UV-light range. In general, UV light can be divided into UV-A radiation (320 - 400 nm) and UV-B radiation (280 - 320 nm). Depending on the position of the absorption maxima, UV filters are divided into UV-A and UV-B filters. In case an UV filter absorbs both, UV-A and UV-B light, it is referred to as a broadband absorber.

Since 2006, the EU commission has recommended that all sunscreen or cosmetic compositions should have an UV-A protection factor, which is at least one third of the labelled sun protection factor (SPF), wherein the sun protection factor refers mainly to the UV-B protection.

2-(4'-Diethylamino-2'-hydroxybenzoyl)benzoic acid hexyl ester is an effective UV-A filter and is used to protect skin from UV radiation (e.g. in sunscreen). 2-(4'-Diethylamino-2'-hydroxybenzoyl)benzoic acid hexyl ester is depicted below, and will in the following also be referred to as compound of formula (I).

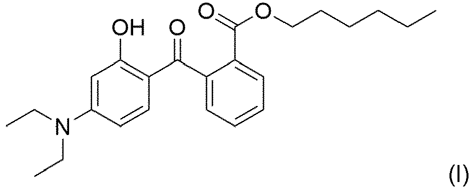

Processes of manufacturing 2-(4'-diethylamino-2'-hydroxybenzoyl)benzoic acid hexyl ester are known from e.g. DE 10011317, EP2155660, and WO 2003097578. In this connection, several challenges have been observed.

During synthesis, the by-product phthalic acid dihexyl ester (also known as PSDHE, dihexylphthalate) is formed. The final product can still contain up to 50-150 ppm PSDHE. According to recent findings, the PSDHE content should be as low as possible, since this substance may according to the harmonised classification and labelling (ATP05) damage fertility and may damage the unborn child.

Further, during the synthesis of 2-(4'-diethylamino-2'-hydroxybenzoyl)benzoic acid hexyl ester process Rhodamine B type dyes (e.g. [9-(2-carboxyphenyl)-6-diethylamino-3-xanthenylidene]-diethylammonium salts) and corresponding esters are formed as impurities, which lead to an undesired discoloration of the final product. In order to purify the final product (less than 10 ppm Rhodamine B type dyes) an additional purification step is required. So far, the purification step comprises dissolution of the contaminated 2-(4'-diethylamino-2'-hydroxybenzoyl)benzoic acid hexyl ester in a nonpolar solvent such as cyclohexane or toluene, adsorption over an activated carbon or silicic acid bed and evaporation of the solvent after passage through the bed.

After the subsequent isolation of the 2-(4'-diethylamino-2'-hydroxybenzoyl)benzoic acid hexyl ester by separating off the toluene and/or 1-hexanol by distillation the clean end product is bottled as melt. The obtained product however still comprises phthalic acid dialkyl ester content in amounts of 15 ppm and more.

Hence, there is an ongoing need for a fast and efficient purification process of 2-(4'-diethylamino-2'-hydroxybenzoyl)benzoic acid hexyl ester. In this connection, it has been an object of the present invention to provide an optimized process for isolating 2-(4'-diethylamino-2'-hydroxybenzoyl)benzoic acid hexyl ester. It has especially been an object to provide a process for purifying 2-(4'-diethylamino-2'-hydroxybenzoyl)benzoic acid hexyl ester, wherein the amount of phthalic acid dihexyl ester is reduced. Finally, it has been an object of the present invention to provide a fast process for purifying 2-(4'-diethylamino-2'-hydroxybenzoyl)benzoic acid hexyl ester, which further provides a safe and convenient to handle final product.

It has surprisingly been found that at least one of the above objects can be achieved by the process according to the present invention. The inventive process provides 2-(4'-diethylamino-2'-hydroxybenzoyl)benzoic acid hexyl ester with low PSDHE content (less than 20 ppm).

In particular, the inventors of the present invention have found a process for effectively purifying the compound of formula (I).

The present invention therefore relates to a process for purifying 2-(4'-diethylamino-2'-hydroxybenzoyl)benzoic acid hexyl ester comprising the steps of
a) subjecting crude 2-(4'-diethylamino-2'-hydroxybenzoyl)benzoic acid hexyl ester to a distillation at a temperature in the range of from 100 to 250 °C at a pressure of less than 10 mbar;
b) isolating 2-(4'-diethylamino-2'-hydroxybenzoyl)benzoic acid hexyl ester as the high boiler.

In the following, preferred embodiments of the process are described in further detail. It is to be understood that each preferred embodiment is relevant on its own as well as in combination with other preferred embodiments.

In a preferred embodiment A1, the crude 2-(4'-diethylamino-2'-hydroxybenzoyl)benzoic acid hexyl ester in step a) comprises 15 to 1000 ppm phthalic acid dihexyl ester.

In a preferred embodiment A2, the phthalic acid dihexyl ester is removed from the crude 2-(4'-diethylamino-2'-hydroxybenzoyl)benzoic acid hexyl ester in step a) as the light boiler.

In a preferred embodiment A3, the distillation in step a) is performed at a temperature in the range of from 160 to 220 °C, preferably in the range of from 190 to 210 °C.

In a preferred embodiment A4, the distillation in step a) is performed at a pressure of from 0.01 to 10 mbar, preferably 0.05 to 5 mbar.

In a preferred embodiment A5, the distillation in step a) is performed at a temperature in the range of from 120 to 180 °C, preferably in the range of from 130 to 170 °C.

In a preferred embodiment A6, the distillation in step a) is performed at a pressure of from 0.0001 to 0.03 mbar, preferably 0.001 to 0.007 mbar.

In a preferred embodiment A7, the process further comprises the steps of
c) collecting the light boiler fraction;
d) subjecting the collected light boiler fraction from step c) to a distillation at a temperature in the range of from 100 to 250 °C at a pressure of less than 10 mbar;
e) isolating 2-(4'-diethylamino-2'-hydroxybenzoyl)benzoic acid hexyl ester as a further fraction of the high boiler.

In a preferred embodiment A8, the collected light boiler fraction from step c) in step d) comprises 50 to 1000 ppm phthalic acid dihexyl ester.

In a preferred embodiment A9, the 2-(4'-diethylamino-2'-hydroxybenzoyl)benzoic acid hexyl ester isolated in step b) comprises less than 20 ppm, preferably less than 15 ppm, more preferably less than 13 ppm, phthalic acid dihexyl ester.

In a preferred embodiment A10, the 2-(4'-diethylamino-2'-hydroxybenzoyl)benzoic acid hexyl ester isolated in step b) has a Gardner value of 9.5 or lower.

In a preferred embodiment A11, the 2-(4'-diethylamino-2'-hydroxybenzoyl)benzoic acid hexyl ester isolated in step b) is obtained with a yield of at least 60 %, preferably at least 80 %, more preferably at least 90 %, even more preferably at least 99 %.

In a preferred embodiment A12, the process is performed as a continuous process with a continuous feed of crude 2-(4'-diethylamino-2'-hydroxybenzoyl)benzoic acid hexyl ester and a continuous removal of the high boiler and light boiler.

In a preferred embodiment A13, the distillation in step a) is performed at a temperature in the range of from more than 180 to 220 °C and at a pressure of from 0.01 to 10 mbar or the distillation in step a) is performed at a temperature in the range of from 120 to 180 °C and at a pressure of from 0.0001 to 0.03 mbar.

### Figures

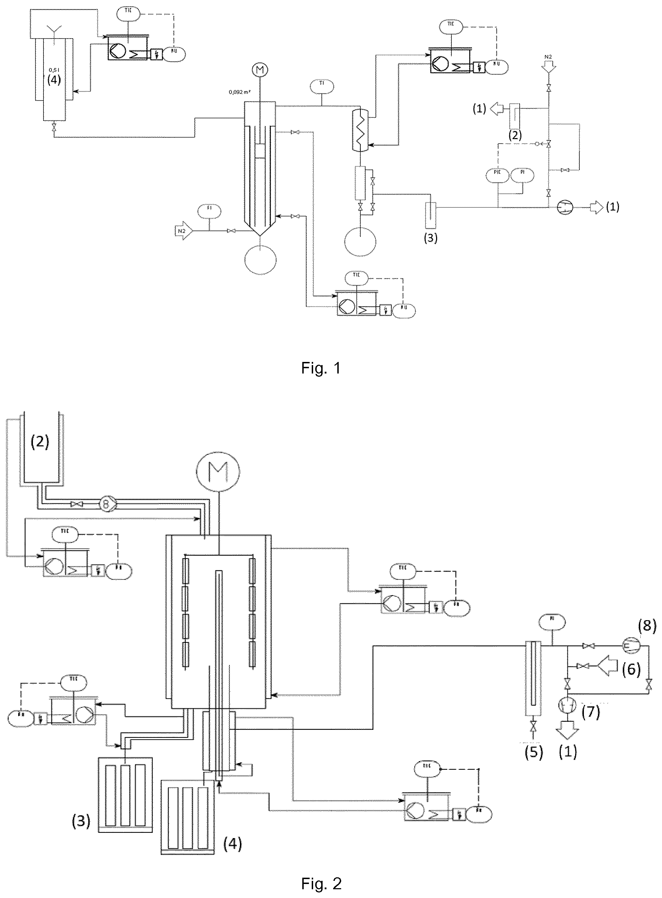

Fig. 1 depicts schematically a thin-film evaporator based process, comprising (1) waste gas, (2) washing bottle, (3) cooling trap, and (4) graduated dosing.
Fig. 2 depicts schematically a short-path evaporator, comprising (1) waste gas, (2) 2L-feed, (3) sump, (4) head, (5) cooling trap, (6) ventilation, (7) rotary vane pump, and (8) oil diffusion pump.

### Detailed Description

Before describing in detail exemplary embodiments of the present invention, definitions important for understanding the present invention are given.

As used in this specification and in the appended claims, the singular forms of "a" and "an" also include the respective plurals unless the context clearly dictates otherwise. In the context of the present invention, the terms "about" and "approximately" denote an interval of accuracy that a person skilled in the art will understand to still ensure the technical effect of the feature in question. The term typically indicates a deviation from the indicated numerical value of ±20 %, preferably ± 15 %, more preferably ± 10 %, and even more preferably ±5 %. It is to be understood that the term "comprising" is not limiting. For the purposes of the present invention the term "consisting of" is considered to be a preferred embodiment of the term "comprising of". If hereinafter a group is defined to comprise at least a certain number of embodiments, this is meant to also encompass a group which preferably consists of these embodiments only. Furthermore, the terms "first", "second", "third" or "(a)", "(b)", "(c)", "(d)" etc. and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein. In case the terms "first", "second", "third" or "(a)", "(b)", "(c)", "(d)", "i", "ii" etc. relate to steps of a method or use or assay there is no time or time interval coherence between the steps, i.e. the steps may be carried out simultaneously or there may be time intervals of seconds, minutes, hours, days, weeks, months or even years between such steps, unless otherwise indicated in the application as set forth herein above or below. It is to be understood that this invention is not limited to the particular methodology, protocols, reagents etc. described herein as these may vary. It is also to be understood that the terminology used herein is for the purpose of describing particular embodiments only, and is not intended to limit the scope of the present invention that will be limited only by the appended claims. Unless defined otherwise, all technical and scientific terms used herein have the same meanings as commonly understood by one of ordinary skill in the art.

The term "compound according to the invention" or "compound of formula (I)" refers to 2-(4'-diethylamino-2'-hydroxybenzoyl)benzoic acid hexyl ester.

The term "alkyl" as used herein denotes in each case a straight-chain or branched alkyl group having usually from 1 to 20 carbon atoms, preferably from 2 to 18 carbon atoms, more preferably 4 to 12 carbon atoms. Examples of an alkyl group are methyl, ethyl, n-propyl, isopropyl, n-butyl, 2-butyl, iso-butyl, tert-butyl, n-pentyl, 1-methylbutyl, 2-methylbutyl, 3 methylbutyl, 2,2-dimethylpropyl, 1 ethylpropyl, 1,1-dimethylpropyl, 1,2-dimethylpropyl, n-hexyl, n-heptyl, n-octyl, n-nonyl, n-decyl, n-undecyl, and n-dodecyl. Iso-butyl and n-hexyl are preferred, in particular n-hexyl.

The term "(Cn-Cm-alkyl)" as used herein denotes in each case a linker moiety, wherein the thereto attached moieties are attached to the terminal carbons.

The term "body-care product" refers to any product suitable to apply to the human body, e.g. sunscreen compositions, bath and shower products, preparations containing fragrances and odoriferous substances, hair-care products, dentifrices, decorative preparations, and cosmetic formulations containing active ingredients. Preferred are sunscreen compositions.

The term "sunscreen composition" or "sunscreen" or "skin-care product" refers to any topical product, which reflects and/or absorbs certain parts of UV radiation. Thus, the term "sunscreen composition" is to be understood as not only including sunscreen compositions, but also any cosmetic compositions that provide UV protection. The term "topical product" refers to a product that is applied to the skin and can refer, e.g., to sprays, lotions, creams, oils, foams, powders, or gels. According to the present invention the sunscreen composition may comprise one or more active agents, e.g., organic UV filters, as well as other ingredients or additives, e.g., emulsifiers, emollients, viscosity regulators, stabilizers, preservatives, or fragrances.

The term "daily care composition" refers to any topical product, which reflects or absorbs certain parts of UV radiation and is used as an everyday care product for the human body, e.g., for face, body or hair. The daily care composition may comprise one or more active agents, e.g., organic or inorganic UV filters, as well as other ingredients or additives, e.g., emulsifiers, emollients, viscosity regulators, stabilizers, preservatives, or fragrances.

Suitable bath and shower additives are, e.g., shower gels, bath-salts, bubble baths and soaps.

Suitable preparations containing fragrances and odoriferous substances are in particular scents, perfumes, toilet waters and shaving lotions (aftershave preparations).

Suitable hair-care products are, e.g., shampoos for humans and animals, in particular dogs, hair conditioners, products for styling and treating hair, perming agents, hair sprays and lacquers, hair gels, hair fixatives and hair dyeing or bleaching agents.

Suitable dentifrices are, e.g., tooth creams, toothpastes, mouth-washes, mouth rinses, antiplaque preparations and cleaning agents for dentures.

Suitable decorative preparations are, e.g., lipsticks, nail varnishes, eye shadows, mascaras, dry and moist make-up, rouge, powders, depilatory agents and suntan lotions.

Suitable cosmetic formulations containing active ingredients are, e.g., hormone preparations, vitamin preparations, vegetable extract preparations and antibacterial preparations.

The cited body-care products can be in the form of creams, ointments, pastes, foams, gels, lotions, powders, make-ups, sprays, sticks or aerosols. They preferably contain the compound of formula (I) in the aqueous phase. Thereby, the compound of formula (I) may acts as a light stabilizer.

The term "photostability" refers to the ability of a UV filter or any other molecule, which is exposed to sunlight, to stay stable upon irradiation. In particular, this means that the compound does not undergo a degradation process upon UV radiation.

The term "critical wavelength" is defined as the wavelength at which the area under the UV protection curve (% protection versus wavelength) represents 90 % of the total area under the curve in the UV region (280-400 nm). For example, a critical wavelength of 370 nm indicates that the protection of the sunscreen composition is not limited to the wavelengths of UV-B, i.e. wavelengths from 280-320 nm, but extends to 370 nm in such a way that 90 % of the total area under the protective curve in the UV region are reached at 370 nm.

The term "ultraviolet filter" or "UV filter" as used herein refers to organic or inorganic compounds, which can absorb and/or reflect UV radiation caused by sunlight. UV filter can be classified based on their UV protection curve as UV-A, UV-B or broadband filters. In the context of the present application, broadband filters may be listed as UV-A filters, as they also provide UV-A protection. In other words, preferred UV-A filters also include broadband filters.

The definition of "broadband" protection (also referred to as broad-spectrum or broad protection) is based on the "critical wavelength". For broadband coverage, UV-B and UV-A protection must be provided. According to the US requirements, a critical wavelength of at least 370 nm is required for achieving broad spectrum protection. Furthermore, it is recommended by the European Commission that all sunscreen or cosmetic compositions should have an UVA protection factor, which is at least one third of the labelled sun protection factor (SPF), e.g. if the sunscreen composition has an SPF of 30 the UVA protection factor has to be at least 10.

Preferred embodiments regarding the process according to the present invention are described hereinafter. It is to be understood that the preferred embodiments of the invention are preferred alone or in combination with each other.

As indicated above, the present invention relates in one embodiment to a process for purifying 2-(4'-diethylamino-2'-hydroxybenzoyl)benzoic acid hexyl ester comprising the steps of
a) subjecting crude 2-(4'-diethylamino-2'-hydroxybenzoyl)benzoic acid hexyl ester to a distillation at a temperature in the range of from 100 to 250 °C at a pressure of less than 10 mbar;
b) isolating 2-(4'-diethylamino-2'-hydroxybenzoyl)benzoic acid hexyl ester as the high boiler.

Preferred embodiments regarding the process are defined hereinafter.

In one embodiment of the present invention, the crude 2-(4'-diethylamino-2'-hydroxybenzoyl)benzoic acid hexyl ester in step a) comprises the impurity phthalic acid dialkyl ester, in particular phthalic acid dihexyl ester.

Preferably, the crude 2-(4'-diethylamino-2'-hydroxybenzoyl)benzoic acid hexyl ester in step a) comprises 15 to 1000 ppm, preferably from 30 to 500 ppm, more preferably from 40 to 450 ppm, even more preferably from 50 to 400 ppm, phthalic acid dialkyl ester.

In this connection, it is to be understood that according to the present invention, phthalic acid dialkyl esters are defined by having the formula (A) 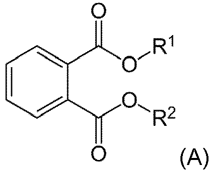 wherein R¹ and R² are C₄-C₁₂-alkyl, preferably wherein R¹ and R² are both n-hexyl.

The phthalic acid dialkyl ester is preferably removed from the crude 2-(4'-diethylamino-2'-hydroxybenzoyl)benzoic acid hexyl ester in step a) as the light boiler.

In this connection, it is noted that removing the phthalic acid dialkyl ester refers to the separation of the impurity phthalic acid dialkyl ester from crude 2-(4'-diethylamino-2'-hydroxybenzoyl)benzoic acid hexyl ester. The separated phthalic acid dialkyl ester may be collected or discarded.

The process according to the present invention provides a light boiler fraction. This light boiler fraction comprises the light boiler (i.e. phthalic acid dialkyl ester, in particular phthalic acid dihexyl ester). The light boiler fraction may further comprise additional impurities such as colored impurities or solvents.

In a preferred embodiment of the present invention, the crude 2-(4'-diethylamino-2'-hydroxybenzoyl)benzoic acid hexyl ester in step a) comprises 15 to 1000 ppm, preferably from 20 to 800 ppm, more preferably from 30 to 600 ppm, even more preferably from 35 to 500 ppm, especially preferred from 40 to 400 ppm, and in particular from 40 to 300 ppm phthalic acid dihexyl ester.

In this connection, the phthalic acid dihexyl ester is preferably removed from the crude 2-(4'-diethylamino-2'-hydroxybenzoyl)benzoic acid hexyl ester in step a) as the light boiler.

In one embodiment of the present invention, the distillation in step a) is performed at a temperature in the range of from 160 to 220 °C, preferably in the range of from 180 to 215 °C, more preferably in the range of from 190 to 210 °C, even more preferably in the range of from 200 to 210 °C.

In one embodiment of the present invention, the distillation in step a) is performed at a pressure of from 0.01 to 10 mbar, preferably from 0.02 to 8 mbar, more preferably from 0.03 to 5 mbar, even more preferably from 0.04 to 3 mbar, and in particular from 0.05 to 2 mbar.

In another embodiment of the present invention, the distillation in step a) is performed at a pressure of from more than 0.01 to less than 10 mbar, preferably from more than 0.02 to less than 8 mbar, more preferably from more than 0.03 to less than 5 mbar, from even more preferably from more than 0.04 to less than 3 mbar, and in particular from more than 0.05 to less than 2 mbar.

In another embodiment of the present invention, the distillation in step a) is performed at a temperature in the range of from 120 to 180 °C, preferably in the range of from 130 to 170 °C, more preferably in the range of from 135 to 150 °C, and in particular in the range of from 140 to 150 °C.

In this connection, it is preferred that the distillation in step a) is performed at a pressure of from 0.0001 to 0.03 mbar, preferably from 0.001 to 0.01, and more preferably from 0.001 to 0.007 mbar.

In a preferred embodiment of the present invention, the distillation in step a) is performed at a temperature in the range of from more than 180 to 220 °C, preferably in the range of from 185 to 215 °C, more preferably in the range of from 190 to 210 °C, even more preferably in the range of from 200 to 210 °C and at a pressure of from 0.01 to 10 mbar, preferably from 0.02 to 8 mbar, more preferably from 0.03 to 5 mbar, even more preferably from 0.04 to 3 mbar, and in particular from 0.05 to 2 mbar; or the distillation in step a) is performed at a temperature in the range of from 120 to 180 °C, preferably in the range of from 130 to 170 °C, more preferably in the range of from 135 to 150 °C, and in particular in the range of from 140 to 150 °C and at a pressure of from 0.0001 to 0.03 mbar, preferably from 0.001 to 0.01, and more preferably from 0.001 to 0.007 mbar.

In one embodiment of the present invention, the process further comprises the steps of
c) collecting the light boiler fraction;
d) subjecting the collected light boiler fraction from step c) to a distillation at a temperature in the range of from 100 to 250 °C at a pressure of less than 10 mbar; and
e) isolating 2-(4'-diethylamino-2'-hydroxybenzoyl)benzoic acid hexyl ester as a further fraction of the high boiler.

In the event that 2-(4'-diethylamino-2'-hydroxybenzoyl)benzoic acid hexyl ester was partly distilled off and is comprised in the light boiling fraction, steps c) to e) provide the possibility to recovery 2-(4'-diethylamino-2'-hydroxybenzoyl)benzoic acid hexyl ester from the collected light boiling fraction.

In one embodiment, the process further comprises the steps of
c) collecting the light boiler fraction;
d) subjecting the collected light boiler fraction from step c) to a distillation at a temperature in the range of from 160 to 220 °C, preferably in the range of from 190 to 210 °C, at a pressure of less than 10 mbar, preferably less than 5 mbar, and in particular less than 0.1 mbar; and
e) isolating 2-(4'-diethylamino-2'-hydroxybenzoyl)benzoic acid hexyl ester as a further fraction of the high boiler.

In one embodiment of the present invention, the collected light boiler fraction from step c) in step d) comprises 50 to 1000 ppm phthalic acid dialkyl ester, in particular phthalic acid dihexyl ester.

According to the present invention, any suitable evaporator may be used.

In one embodiment of the present invention, a thin-film evaporator is used. Thin-film evaporators (also known as wiped-film evaporator) or a short-path evaporator may be named.

A short-path evaporator provides for a continuous distillation process, a very low operating pressure, a short residence time, a high evaporation rate, a low fouling on evaporator wall, and/or a compact design.

In one embodiment of the present invention, the 2-(4'-diethylamino-2'-hydroxybenzoyl)benzoic acid hexyl ester isolated in step b) comprises less than 20 ppm, preferably less than 15 ppm, more preferably less than 13 ppm, even more preferable less than 10 ppm, and in particular less than 5 ppm, phthalic acid dialkyl ester. Preferably, the 2-(4'-diethylamino-2'-hydroxybenzoyl)benzoic acid hexyl ester isolated in step b) comprises less than 20 ppm, preferably less than 15 ppm, more preferably less than 13 ppm, even more preferable less than 10 ppm, and in particular less than 5 ppm, phthalic acid dihexyl ester.

In a particular embodiment of the present invention, the 2-(4'-diethylamino-2'-hydroxybenzoyl)benzoic acid hexyl ester isolated in step b) is free of phthalic acid dialkyl ester, in particular free of phthalic acid dihexyl ester.

In one embodiment of the present invention, the crude 2-(4'-diethylamino-2'-hydroxybenzoyl)benzoic acid hexyl ester in step a) has a Gardner Value of 8.2 or more.

In one embodiment of the present invention, the 2-(4'-diethylamino-2'-hydroxybenzoyl)benzoic acid hexyl ester isolated in step b) has a Gardner value of 9.5 or lower, preferably of 9 or lower, more preferably of 8.5 or lower, even more preferably of 8.2 or lower, especially preferred of 8.0 and in particular of 7.5 or lower.

In one embodiment of the present invention, the 2-(4'-diethylamino-2'-hydroxybenzoyl)benzoic acid hexyl ester isolated in step b) comprises less than 20 ppm, preferably less than 15 ppm, more preferably less than 13 ppm, even more preferable less than10 ppm, and in particular less than 5 ppm, phthalic acid dialkyl ester and has a Gardner value of 9.5 or lower, preferably of 9 or lower, more preferably of 8.5 or lower, even more preferably of 8.2 or lower, especially preferred of 8.0 and in particular of 7.5 or lower.

In a preferred embodiment of the present invention, the 2-(4'-diethylamino-2'-hydroxybenzoyl)benzoic acid hexyl ester isolated in step b) comprises less than 20 ppm, preferably less than 15 ppm, more preferably less than 13 ppm, even more preferable less than 10 ppm, and in particular less than 5 ppm, phthalic acid dihexyl ester and has a Gardner value of 9.5 or lower, preferably of 9 or lower, more preferably of 8.5 or lower, even more preferably of 8.2 or lower, especially preferred of 8.0 and in particular of 7.5 or lower.

In one embodiment of the present invention, the 2-(4'-diethylamino-2'-hydroxybenzoyl)benzoic acid hexyl ester isolated in step b) is obtained with a yield of at least 60 %, preferably at least 80 %, more preferably at least 90 %, even more preferably at least 99 %.

In one embodiment of the present invention, the 2-(4'-diethylamino-2'-hydroxybenzoyl)benzoic acid hexyl ester isolated in step b) is obtained with a yield of at least 90 %, preferably at least 95 %, more preferably at least 99 %, comprises less than 20 ppm, preferably less than 15 ppm, more preferably less than 13 ppm, even more preferable less than 10 ppm, and in particular less than 5 ppm, phthalic acid dihexyl ester and having a Gardner value of 9.5 or lower, preferably of 9 or lower.

In one embodiment of the present invention, the 2-(4'-diethylamino-2'-hydroxybenzoyl)benzoic acid hexyl ester isolated in step b) is obtained with a yield of at least 60 %, preferably at least 65 %, more preferably at least 70 %, comprises less than 20 ppm, preferably less than 15 ppm, more preferably less than 13 ppm, even more preferable less than 10 ppm, and in particular less than 5 ppm, phthalic acid dihexyl ester and having a Gardner value of 9 or lower, preferably of 8.2 or lower, more preferably 8.0 or lower, and in particular 7.5 or lower.

In one embodiment of the present invention, the process is performed as a continuous process with a continuous feed of at least 50 g, preferably at least 80 g, crude 2-(4'-diethylamino-2'-hydroxybenzoyl)benzoic acid hexyl ester per hour and a continuous removal of the high boiler and light boiler.

In one embodiment, the present invention relates to a body-care product or a daily care composition comprising the 2-(4'-diethylamino-2'-hydroxybenzoyl)benzoic acid hexyl ester obtained according to the claimed process. In particular, the present invention relates to a sunscreen composition comprising the 2-(4'-diethylamino-2'-hydroxybenzoyl)benzoic acid hexyl ester obtained according to the claimed process.

In one embodiment, the present invention relates to the use of the 2-(4'-diethylamino-2'-hydroxybenzoyl)benzoic acid hexyl ester obtained according to the claimed process as UV filter in a body-care product or a daily care composition, in particular in a sunscreen composition.

The present invention is further illustrated by the following examples.

### Examples

### Methods

HPLC method for determination of 2-(4'-diethylamino-2'-hydroxybenzoyl)benzoic acid hexyl ester, phthalate esters, and rhodamine type colored compounds have been performed by standard state of the art methods.

The PSDHE content of Ex. 11, 14A, and 14B was measured by HPLC Analysis using a UPLC Agilent 1290 Infinity.

The PSDHE content of Ex. 1 to 10, 12, 13, and 15 was measured by GC Analysis using a Agilent 6890.

The Gardner value has been performed by standard state of the art methods using a spectral colorimeter LICO 500 from Hach Lange by heating the sample to 75 °C and after cooling of 5-7 min at room temperature.

Analytical standards for calibration have been performed by standard state of the art methods.

The crude 2-(4'-diethylamino-2'-hydroxybenzoyl)benzoic acid hexyl ester for Examples 1 to 14B was obtained according to EP 1 506 159, example 3, except that the product was not drawn off as a melt but treated further as outlined below.

Inventive Examples 1 to 3 and Comparative Example 8 were fed with a crude 2-(4'-diethylamino-2'-hydroxybenzoyl)benzoic acid hexyl ester having a PSDHE content of 78 ppm and a Gardner of 7.5.

Inventive Examples 4 to 7 were fed with a crude 2-(4'-diethylamino-2'-hydroxybenzoyl)benzoic acid hexyl ester having a PSDHE content of 68 ppm and a Gardner of 7.5.

Inventive Examples 9 to 14B were fed with a crude 2-(4'-diethylamino-2'-hydroxybenzoyl)benzoic acid hexyl ester having a PSDHE content of 82 ppm and a Gardner of 7.5.

Inventive Examples 1 to 7 were performed in a wiped-film evaporator (Sambay type, evaporation area 920cm²).

The crude 2-(4'-diethylamino-2'-hydroxybenzoyl)benzoic acid hexyl ester (crude UVA+) was fed to the thin-film evaporator and the purification was performed under the conditions according to Table 1. The analytical results are summarized in Table 2.

For Comparative Example 8 water vapor stripping was used.

**Table 1: Conditions for Sambay thin-film evaporator and water vapor stripping and the remaining PSDHE content in the high boiler fraction.**

| Ex. | T [°C] | p [mbar] | feed [g/h] |
|---|---|---|---|
| 1 | 220 | 0.1-0.03 | 100 |
| 2 | 160-180 | 0.1-0.05 | 100 |
| 3 | 220 | 0.1-0.03 | 100-150 |
| 4 | 180 | 0.25 | 150-200 |
| 5 | 190 | 0.013 | 100 |
| 6 | 200 | 0.07 | 100 |
| 7 | 210 | 0.05 | 100 |
| 8 | 60 | 2 | |

**Table 2: Analytic summary for Sambay thin-film evaporator and water vapor stripping. n.d. denotes "not detected". The Gardner relates to the value obtained of the product after thin-film evaporator.**

| Ex. | PSDHE [ppm] | Depletion PSDHE [ppm] | Yield UVA+ [%] | Gardner | Delta Gardner |
|---|---|---|---|---|---|
| 1 | <15 | up to 78 | 68.96 | n.d. | n.d. |
| 2 | 57 | 21 | 99.35 | n.d. | n.d. |
| 3 | <15 | up to 78 | 63.22 | 9.3 | 1.8 |
| 4 | 58 | 10 | 99.98 | 8 | 0.5 |
| 5 | 34 | 34 | 99.94 | 8.3 | 0.8 |
| 6 | <15 | up to 68 | 99.94 | 8.7 | 1.2 |
| 7 | <15 | up to 68 | 99.92 | 9.4 | 1.9 |
| 8 | 77 | 1 | 95.05 | 7.5 | 0 |

Inventive Examples 9 to 15 were performed in a short-path evaporator (wiped-film evaporator, KDL-5 from company UIC).

The crude 2-(4'-diethylamino-2'-hydroxybenzoyl)benzoic acid hexyl ester was fed to the short-path evaporator and the purification was performed under the conditions according to Table 3. The analytical results are summarized in Table 4.

The light boiling fractions of Inventive Examples 14A and 14B were collected and combined. The combined light boiling fraction was again subjected to the purification process (Inventive Example 15).

Respectively, Inventive Example 15 was fed with a crude 2-(4'-diethylamino-2'-hydroxybenzoyl)benzoic acid hexyl ester having a PSDHE content of 482 ppm and a Gardner of 6.9.

**Table 3: Conditions for short-path evaporator and the remaining PSDHE content in the high boiler fraction.**

| Ex. | T [°C] | p [mbar] | feed [g/h] | rpm |
|---|---|---|---|---|
| 9 | 145 | 0.001 | 150 | 170 |
| 10 | 145 | 0.001 | 80 | 200 |
| 11 | 145 | 0.001 | 150 | 200 |
| 12 | 130 | 0.001 | 115 | 200 |
| 13 | 145 | 0.006 | 120 | 200 |
| 14A | 145 | 0.001 | 120 | 200 |
| 14B | 145 | 0.001 | 120 | 200 |
| 15 | 145 | 0.001 | 120 | 200 |

**Table 4: Analytic summary for short-path evaporator. N.d. denotes "not detected". The Gardner relates to the value obtained of the product after thin-film evaporator.**

| Ex. | PSDHE [ppm] | Depletion PSDHE [ppm] | Yield UVA+ [%] | Gardner | Delta Gardner |
|---|---|---|---|---|---|
| 9 | 20 | 62 | 92.50 | 7.9 | 0.6 |
| 10 | 11 | up to 82 | 74.08 | 7.7 | 0.3 |
| 11 | 9.9 | 72.1 | 82.48 | 7.3 | 0.3 |
| 12 | 34 | 48 | 94.00 | 7.2 | 0.2 |
| 13 | <15 | up to 82 | 82.63 | 7.5 | 0.5 |
| 14A | 9.5 | 73.5 | 84.80 | 7.3 | 0.3 |
| 14B | 12.0 | 70 | 84.80 | 7.0 | 0.0 |
| 15 | 49 | 433 | 82.03 | 7.4 | 0.5 |

As can be seen from Tables 1 to 4, all Inventive Examples provide for a reduced PSDHE content in the final 2-(4'-diethylamino-2'-hydroxybenzoyl)benzoic acid hexyl ester. Further, all Inventive Examples provide an improved reduced PSDHE content in the final 2-(4'-diethylamino-2'-hydroxybenzoyl)benzoic acid hexyl ester when compared to Comparative Example 8.

## Claims

1. A process for purifying 2-(4'-diethylamino-2'-hydroxybenzoyl)benzoic acid hexyl ester comprising the steps of
a) subjecting crude 2-(4'-diethylamino-2'-hydroxybenzoyl)benzoic acid hexyl ester to a distillation at a temperature in the range of from 100 to 250 °C at a pressure of less than 10 mbar;
b) isolating 2-(4'-diethylamino-2'-hydroxybenzoyl)benzoic acid hexyl ester as the high boiler.

2. The process according to claim 1, wherein the crude 2-(4'-diethylamino-2'-hydroxybenzoyl)benzoic acid hexyl ester in step a) comprises 15 to 1000 ppm phthalic acid dihexyl ester.

3. The process according to claim 1 or 2, wherein the phthalic acid dihexyl ester is removed from the crude 2-(4'-diethylamino-2'-hydroxybenzoyl)benzoic acid hexyl ester in step a) as the light boiler.

4. The process according to any one of claims 1 to 3, wherein the distillation in step a) is performed at a temperature in the range of from 160 to 220 °C, preferably in the range of from 190 to 210 °C.

5. The process according to any one of claims 1 to 4, wherein the distillation in step a) is performed at a pressure of from 0.01 to 10 mbar, preferably 0.05 to 5 mbar.

6. The process according to any one of claims 1 to 3, wherein the distillation in step a) is performed at a temperature in the range of from 120 to 180 °C, preferably in the range of from 130 to 170 °C.

7. The process according to any one of claims 1 to 3 or 6, wherein the distillation in step a) is performed at a pressure of from 0.0001 to 0.03 mbar, preferably 0.001 to 0.007 mbar.

8. The process according to any one of claims 1 to 3, wherein the distillation in step a) is performed at a temperature in the range of from more than 180 to 220 °C and at a pressure of from 0.01 to 10 mbar or
wherein the distillation in step a) is performed at a temperature in the range of from 120 to 180 °C and at a pressure of from 0.0001 to 0.03 mbar.

9. The process according to any one of claims 1 to 3, or 6 to 8, further comprising the steps of
c) collecting the light boiler fraction;
d) subjecting the collected light boiler fraction from step c) to a distillation at a temperature in the range of from 100 to 250 °C at a pressure of less than 10 mbar; and
e) isolating 2-(4'-diethylamino-2'-hydroxybenzoyl)benzoic acid hexyl ester as a further fraction of the high boiler.

10. The process according to any one of claims 1 to 3 or 6 to 9, wherein the collected light boiler fraction from step c) in step d) comprises 50 to 1000 ppm phthalic acid dihexyl ester.

11. The process according to any one of claims 1 to 10, wherein the 2-(4'-diethylamino-2'-hydroxybenzoyl)benzoic acid hexyl ester isolated in step b) comprises less than 20 ppm, preferably less than 15 ppm, more preferably less than 13 ppm, phthalic acid dihexyl ester.

12. The process according to any one of claims 1 to 11, wherein the 2-(4'-diethylamino-2'-hydroxybenzoyl)benzoic acid hexyl ester isolated in step b) has a Gardner value of 9.5 or lower.

13. The process according to any one of claims 1 to 12, wherein the 2-(4'-diethylamino-2'-hydroxybenzoyl)benzoic acid hexyl ester isolated in step b) is obtained with a yield of at least 60 %, preferably at least 80 %, more preferably at least 90 %, even more preferably at least 99 %.

14. The process according to any one of claims 1 to 13, wherein the process is performed as a continuous process with a continuous feed of crude 2-(4'-diethylamino-2'-hydroxybenzoyl)benzoic acid hexyl ester and a continuous removal of the high boiler and light boiler.

## Patentansprüche

1. Verfahren zur Reinigung von 2-(4'-Diethylamino-2'-hydroxybenzoyl)benzoesäurehexylester, umfassend die Schritte
a) Destillieren von rohem 2-(4'-Diethylamino-2'-hydroxybenzoyl)benzoesäurehexylester bei einer Temperatur im Bereich von 100 bis 250 °C bei einem Druck von weniger als 10 mbar;
b) Isolieren von 2-(4'-Diethylamino-2'-hydroxybenzoyl)benzoesäurehexylester als Hochsieder.

2. Verfahren nach Anspruch 1, wobei der rohe 2-(4'-Diethylamino-2'-hydroxybenzoyl)benzoesäurehexylester in Schritt a) 15 bis 1000 ppm Phthalsäuredihexylester enthält.

3. Verfahren nach Anspruch 1 oder 2, wobei der Phthalsäuredihexylester in Schritt a) als Leichtsieder aus dem rohen 2-(4'-Diethylamino-2'-hydroxybenzoyl)benzoesäurehexylester entfernt wird.

4. Verfahren nach einem der Ansprüche 1 bis 3, wobei die Destillation in Schritt a) bei einer Temperatur im Bereich von 160 bis 220 °C, vorzugsweise im Bereich von 190 bis 210 °C, durchgeführt wird.

5. Verfahren nach einem der Ansprüche 1 bis 4, wobei die Destillation in Schritt a) bei einem Druck von 0,01 bis 10 mbar, vorzugsweise 0,05 bis 5 mbar, durchgeführt wird.

6. Verfahren nach einem der Ansprüche 1 bis 3, wobei die Destillation in Schritt a) bei einer Temperatur im Bereich von 120 bis 180 °C, vorzugsweise im Bereich von 130 bis 170 °C, durchgeführt wird.

7. Verfahren nach einem der Ansprüche 1 bis 3 oder 6, wobei die Destillation in Schritt a) bei einem Druck von 0,0001 bis 0,03 mbar, vorzugsweise 0,001 bis 0,007 mbar, durchgeführt wird.

8. Verfahren nach einem der Ansprüche 1 bis 3, wobei die Destillation in Schritt a) bei einer Temperatur im Bereich von mehr als 180 bis 220 °C und bei einem Druck von 0,01 bis 10 mbar durchgeführt wird oder
wobei die Destillation in Schritt a) bei einer Temperatur im Bereich von 120 bis 180 °C und einem Druck von 0,0001 bis 0,03 mbar durchgeführt wird.

9. Verfahren nach einem der Ansprüche 1 bis 3 oder 6 bis 8, ferner umfassend die Schritte
c) Sammeln der Leichtsiederfraktion;
d) Destillieren der gesammelten Leichtsiederfraktion aus Schritt c) bei einer Temperatur im Bereich von 100 bis 250 °C bei einem Druck von weniger als 10 mbar; und
e) Isolieren von 2-(4'-Diethylamino-2'-hydroxybenzoyl)benzoesäurehexylester als weitere Fraktion des Hochsieders.

10. Verfahren nach einem der Ansprüche 1 bis 3 oder 6 bis 9, wobei die gesammelte Leichtsiederfraktion aus Schritt c) in Schritt d) 50 bis 1000 ppm Phthalsäuredihexylester enthält.

11. Verfahren nach einem der Ansprüche 1 bis 10, wobei der in Schritt b) isolierte 2-(4'-Diethylamino-2'-hydroxybenzoyl)benzoesäurehexylester weniger als 20 ppm, bevorzugt weniger als 15 ppm, besonders bevorzugt weniger als 13 ppm, Phthalsäuredihexylester enthält.

12. Verfahren nach einem der Ansprüche 1 bis 11, wobei der in Schritt b) isolierte 2-(4'-Diethylamino-2'-hydroxybenzoyl)benzoesäurehexylester einen Gardner-Wert von 9,5 oder weniger aufweist.

13. Verfahren nach einem der Ansprüche 1 bis 12, wobei der in Schritt b) isolierte 2-(4'-Diethylamino-2'-hydroxybenzoyl)benzoesäurehexylester mit einer Ausbeute von mindestens 60 %, bevorzugt mindestens 80 %, weiter bevorzugt mindestens 90 %, noch weiter bevorzugt mindestens 99 %, erhalten wird.

14. Verfahren nach einem der Ansprüche 1 bis 13, **dadurch gekennzeichnet, dass** das Verfahren als kontinuierliches Verfahren mit kontinuierlicher Zufuhr von rohem 2-(4'-Diethylamino-2'-hydroxybenzoyl)benzoesäurehexylester und kontinuierlicher Entfernung des Hochsieders und Leichtsieders durchgeführt wird.

## Revendications

1. Procédé de purification de l'ester hexylique de l'acide 2-(4'-diéthylamino-2'-hydroxybenzoyl)benzoïque comprenant les étapes de
a) soumission de l'ester hexylique de l'acide 2-(4'-diéthylamino-2'-hydroxybenzoyl)benzoïque brut à une distillation à une température dans la plage de 100 à 250 °C et à une pression inférieure à 10 mbars ;
b) isolement de l'ester hexylique de l'acide 2-(4-diéthylamino-2'-hydroxybenzoyl)benzoïque comme composé à point d'ébullition élevé.

2. Procédé selon la revendication 1, dans lequel l'ester hexylique de l'acide 2-(4'-diéthylamino-2'-hydroxybenzoyl)benzoïque brut dans l'étape a) comprend 15 à 1 000 ppm d'ester dihexylique de l'acide phtalique.

3. Procédé selon la revendication 1 ou 2, dans lequel l'ester dihexylique de l'acide phtalique est éliminé de l'ester hexylique de l'acide 2-(4'-diéthylamino-2'-hydroxybenzoyl)benzoïque brut dans l'étape a) en tant que composé à bas point d'ébullition.

4. Procédé selon l'une quelconque des revendications 1 à 3, dans lequel la distillation dans l'étape a) est réalisée à une température dans la plage allant de 160 à 220 °C, préférablement dans la plage allant de 190 à 210 °C.

5. Procédé selon l'une quelconque des revendications 1 à 4, dans lequel la distillation dans l'étape a) est effectuée à une pression de 0,01 à 10 mbars, de préférence de 0,05 à 5 mbars.

6. Procédé selon l'une quelconque des revendications 1 à 3, dans lequel la distillation dans l'étape a) est réalisée à une température dans la plage allant de 120 à 180 °C, préférablement dans la plage allant de 130 à 170 °C.

7. Procédé selon l'une quelconque des revendications 1 à 3 ou 6, dans lequel la distillation dans l'étape a) est effectuée à une pression de 0,0001 à 0,03 mbar, de préférence de 0,001 à 0,007 mbar.

8. Procédé selon l'une quelconque des revendications 1 à 3, dans lequel la distillation dans l'étape a) est effectuée à une température dans la plage de plus de 180 à 220 °C et à une pression de 0,01 à 10 mbars ou
dans lequel la distillation de l'étape a) est effectuée à une température dans la plage de 120 à 180 °C et à une pression dans la plage de 0,0001 à 0,03 mbar.

9. Procédé selon l'une quelconque des revendications 1 à 3, ou 6 à 8, comprenant en outre les étapes de :
c) collecte de la fraction à bas point d'ébullition ;
d) soumission de la fraction à bas point d'ébullition collectée de l'étape c) à une distillation à une température dans la plage de 100 à 250 °C à une pression inférieure à 10 mbars ; et
e) isolement de l'ester hexylique de l'acide 2-(4-diéthylamino-2'-hydroxybenzoyl)benzoïque comme fraction supplémentaire du composé à haut point d'ébullition.

10. Procédé selon l'une quelconque des revendications 1 à 3 ou 6 à 9, dans lequel la fraction de composé à bas point d'ébullition collectée de l'étape c) dans l'étape d) comprend 50 à 1 000 ppm d'ester dihexylique de l'acide phtalique.

11. Procédé selon l'une quelconque des revendications 1 à 10, dans lequel l'ester hexylique de l'acide 2-(4'-diéthylamino-2'-hydroxybenzoyl)benzoïque isolé dans l'étape b) comprend moins de 20 ppm, de préférence moins de 15 ppm, plus préférablement moins de 13 ppm, d'ester dihexylique de l'acide phtalique.

12. Procédé selon l'une quelconque des revendications 1 à 11, dans lequel l'ester hexylique de l'acide 2- (4'-diéthylamino-2'-hydroxybenzoyl)benzoïque isolé dans l'étape b) a une valeur Gardner de 9,5 ou moins.

13. Procédé selon l'une quelconque des revendications 1 à 12, dans lequel l'ester hexylique de l'acide 2-(4'-diéthylamino-2'-hydroxybenzoyl)benzoïque isolé dans l'étape b) est obtenu avec un rendement d'au moins 60 %, de préférence d'au moins 80 %, plus préférablement d'au moins 90 %, encore plus préférablement d'au moins 99 %.

14. Procédé selon l'une quelconque des revendications 1 à 13, dans lequel le procédé est mis en œuvre sous la forme d'un procédé continu avec une alimentation continue d'ester hexylique d'acide 2-(4'-diéthylamino-2'-hydroxybenzoyl)benzoïque brut et une élimination en continu du composé à haut point d'ébullition et du composé à bas point d'ébullition.
